# EUROPEAN PATENT APPLICATION

(11) **EP 3 744 378 A1**
(43) Date of publication of application: **02.12.2020**
(21) Application number: 18901942.5
(22) Date of filing: 25.01.2018
(51) Int. Cl.: A61M 25/00, A61M 25/098

(54) **CATHETER**

(71) Applicant: ASAHI INTECC CO., LTD., Seto-shi, Aichi 489-0071 (JP)
(72) Inventor: KATSURADA Takeharu, Seto-shi, Aichi 489-0071 (JP); OGIDO Moritaka, Seto-shi, Aichi 489-0071 (JP)
(74) Representative: Winter, Brandl, Fürniss, Hübner, Röss, Kaiser, Polte - Partnerschaft mbB
(86) International application number: PCT/JP2018/002180
(87) International publication number: WO 2019/146026

(57) **Abstract**

[Problem to be Solved]

To provide a catheter capable of preventing collapse of the catheter tip even when inserted into a stenosis such as CTO or the like, and being inserted easily into the stenosis.

[Solution to Problem]

A catheter 1 comprises a catheter body 5, and a distal tip 3 joined to a distal end of the catheter body 5 and made of a resin including tungsten powder 2, wherein the distal tip 3 includes a distal region 7 and a proximal region 9 disposed in a proximal side of the distal region 7, and a hardness of the distal region 7 is higher than a hardness of the proximal region 9. Thus, the proximal region 9 has a followability to a medical device such as a guidewire and the like used together, the catheter 1 has an effect of improving insertability into the stenosis and an effect of preventing the collapse of the distal tip 3 of the catheter 1 when the catheter 1 is inserted into the stenosis.

## Description

### Technical Field

The present invention relates to a catheter used by inserting into a lumen of a blood vessel or the like of a patient.

### Background Art

A catheter used by inserting into a lumen of a blood vessel or the like of a patient is known conventionally. A physician performing a procedure inserts a guidewire into the lumen of the catheter, protrudes a distal end of the guidewire from a distal end of the catheter, gets the catheter to a lesion by leading of the guide wire, places a device such as a stent or an embolic coil in the lesion through the lumen of the catheter.

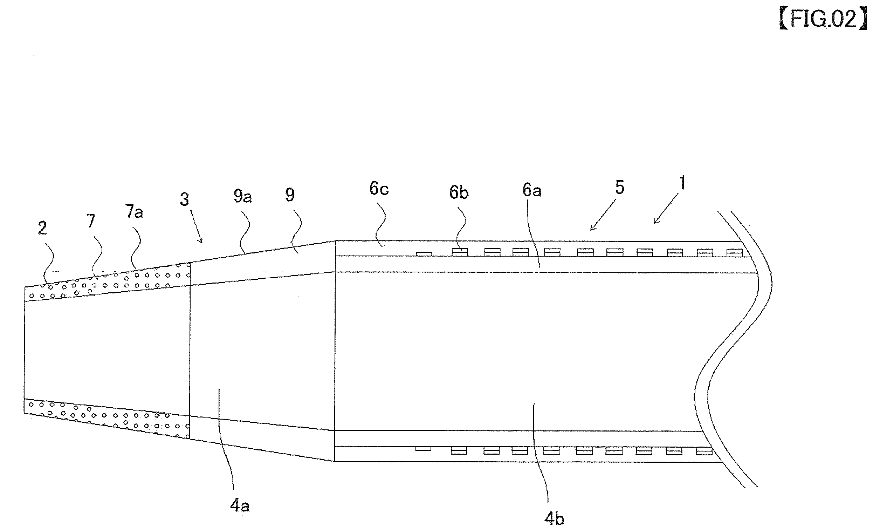

A distal portion of the catheter used in the procedure is generally configured to be soft in order to improve a tracking performance to the guide wire, or to reduce a risk of perforating the lumen of the blood vessel or the like. For example, Patent Document 1, lower the flexural modulus of the distal end, it is raised catheter tube a flexural modulus of the proximal end has been described (see FIG. 2, etc.).

### [Prior art documents]

### [Patent Document]

Patent Document 1 : Japanese Patent Application Publication No. 8(1996)-57035 A

### Summary of the Invention

### Technical Problems

A procedure for severe patients having CTO (Chronic Total Occlusion) or the lesion close to CTO is expected recently, however, we have a problem that the conventional catheter described above can not be inserted into such lesions well.

The present invention has been made in response to forgoing problems of the convetional technique, and is intended to provide a catheter capable of preventing collapse of the catheter tip even when inserted into a stenosis such as CTO or the like, and being inserted easily into the stenosis. Solution for Problems

It is characterized in that, to solve the foregoing problems, a catheter according to a first aspect of the present invention comprises a main body and a distal portion joined to a distal end of the main body, and made of a resin including metal or metal compound, wherein the distal portion includes a distal region and a proximal region disposed in a proximal side of the distal region, and a hardness of the distal region is higher than a hardness of the proximal region.

A second aspect of the present invention is characterized in that, in the catheter according to the first aspect, a surface roughness of the distal region is greater than a surface roughness of the proximal region.

A third aspect of the present invention is characterized in that, in the catheter according to the first aspect or the second aspect, degree of exposure of metal or metal compound on the surface of the distal region is higher than degree of exposure of metal or metal compound on the surface of the proximal region.

Furthermore, a fourth aspect of the present invention is characterized in that, in the catheter according to any one of the first aspect to the third aspect, metal or metal compound includes radiopaque metal. Advantageous Effects of Invention

According to the first aspect of the present invention, as the catheter comprises a main body, and a distal portion joined to a distal end of the main body and made of a resin including metal or metal compound, wherein the distal portion includes a distal region and a proximal region disposed in a proximal side of the distal region, and a hardness of the distal region is higher than a hardness of the proximal region, the proximal region has a followability to a medical device such as a guidewire used together, and the catheter has an effect of improving insertability into the stenosis and an effect of preventing the collapse of the distal portion of the catheter when the catheter is inserted into the stenosis.

According to the second aspect of the present invention, as a surface roughness of the distal region is greater than a surface roughness of the proximal region in the catheter of the first aspect of the present invention, in addition to the effects of the catheter of the first aspect of the present invention, it is capable of further improving insertability into the stenosis to reduce the contact resistance between hard distal region and the stenosis.

According to the third aspect of the present invention, as degree of exposure of metal or metal compound on the surface of the distal region is higher than degree of exposure of metal or metal compound on the surface of the proximal region in the catheter of the first aspect or the second aspect of the present invention, in addition to the effects of the catheter of the first aspect or the second aspect of the present invention, it is capable of improving insertability into the stenosis to reduce the contact resistance between the distal region and the stenosis by utilizing metal or metal compound contained.

Further, according to the fourth aspect of the present invention, as metal or metal compound includes radiopaque metal in the catheter of any one of the first aspect to the third aspect of the present invention, in addition to the effects of the catheter of any one of the first aspect to the third aspect of the present invention, it is capable of improving a visibility of the distal portion by adding a perforamance of a visibility.

### BRIEF DESCRIPTION OF THE DRAWINGS

FIG.1 is an overall view of the catheter of a first embodiment of the present invention.
FIG. 2 is a longitudinal sectional view around a distal portion of the catheter of the first embodiment.
FIG. 3 is a longitudinal sectional view around a distal portion of the catheter of the second embodiment.
FIG. 4 is a longitudinal sectional view around a distal portion of the catheter of the third embodiment.
FIG. 5 is a longitudinal sectional view around a distal portion of the catheter of the fourth embodiment.
FIG. 6 is a longitudinal sectional view around a distal portion of the catheter of the fifth embodiment.

### Description of Embodiments

Hereinafter, embodiments of the present invention will be described with reference to the drawings.

### (First Embodiment)

First of all, a first embodiment of the present invention will be described. The drawings used in the present embodiment is exaggerated to facilitate understanding of the present invention, the dimensions of the drawings differ from actual dimensions.

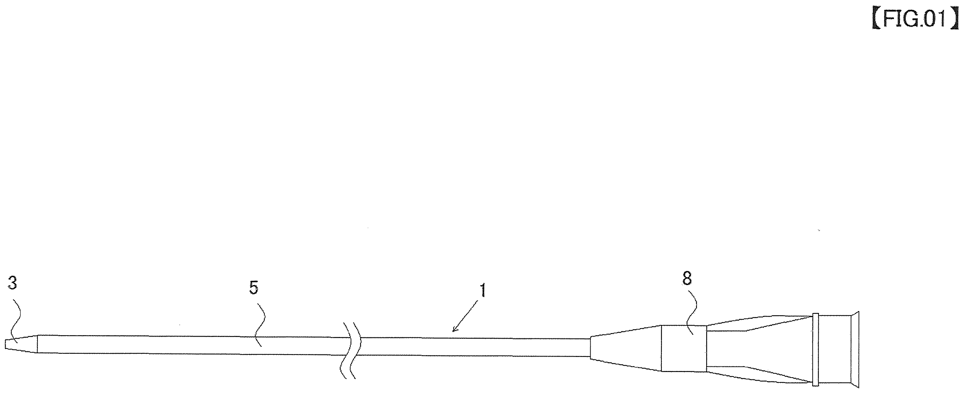

FIG.1 is an overall view of the catheter of a first embodiment of the present invention, FIG. 2 is a longitudinal sectional view around a distal portion of the catheter of the first embodiment.

As shown in FIG. 1, a catheter 1 of the present embodiment includes a catheter body 5 (corresponding to "a main body" of the present invention), a distal tip 3 (corresponding to "a distal portion" of the present invention) joined to a distal end of the catheter body 5, and a connector 8 joined to a proximal end of the catheter body 5.

The catheter body 5 includes an inner layer 6a, a braid 6b wound around an outer periphery of the inner layer 6a, and an outer layer 6c covering the inner layer 6a and the braid 6b as shown in FIG. 2.

The inner layer 6a is an elongated hollow tubular body made of a resin and forms a lumen 4b for inserting a guidewire or other medical devices inside. Resin material forming the inner layer 6a is not particularly limited, PTFE (polytetrafluoroethylene) is used in the present embodiment.

The braid 6b is formed by weaving a total of 24 element wires of twelve first wires and twelve second wires (12 × 12) into a mesh alternately. The material constituting the braid 6b is also not particularly limited, a stainless alloy is used in the present embodiment.

The outer layer 6c is made of resin and covers the inner layer 6a and the braid 6b. Resin material forming the outer layer 6c is not particularly limited, polyamide, polyamide elastomer, polyester, polyurethane and the like can be used, the polyamide is used in the present embodiment.

The distal tip 3 is joined to the distal end of the catheter body 5 has a tubular shape with a lumen 4a communicating with the lumen 4b of the catheter body 5, and tapered periphery gradually decreasing diameter toward the distal end of the catheter body 5.

Further, the distal tip 3 includes a distal region 7 located on a distal side of the distal tip 3 and a proximal region 9 located on a proximal side of the distal region 7.

Resin material forming the distal tip 3 is also not particularly limited, polyurethane elastomer is used in the present embodiment.

Powder made of biocompatible metal or metal compound is mixed inside the distal tip 3, tungsten powder 2 is mixed inside the distal tip 3 in the present embodiment.

Incidentally, material mixed inside the distal tip 3 is not limited to tungsten powder, it may be powder made of radiopaque metal or radiopaque metal compound such as gold, platinum, iridium, platinum-iridium alloys, barium, barium sulfate, bismuth, bismuth compounds (bismuth oxide, bismuth trioxide, bismuth subcarbonate, bismuth subcarbonate, tungsten bismuth), zirconium oxide, tantalum, cobalt chrome alloys, tungsten, tungsten-based compound (tungsten oxide, tungsten dioxide, tungsten trioxide), stainless steel, or titanium. It may also be powder made of radiolucent metal or radiolucent metal compound.

However, if powder consisting of radiopaque metal is mixed inside the distal tip 3, visibility of the distal tip 3 under X-ray fluoroscopy can be enhanced.

This point is similar with respect to powder of biocompatible metal or metal compound mixed inside the distal tip 3 in embodiments described below.

In the present embodiment, tungsten powder 2 is mixed only in the distal region 7, not mixed in the proximal region 9. Thus, in the present embodiment, the hardness of the distal region 7 is set higher than the hardness of the proximal region 9. In addition, an outer surface 7a of the distal region 7 and an outer surface 9a of the proximal region 9 are smooth in the present embodiment.

The connector 8 is also made of resin, has a lumen communicating with the lumen 4b of the catheter body 5, and is joined to the proximal end of the catheter body 5.

According to the catheter 1 of the present embodiment, as the catheter 1 comprises a catheter body 5, and a distal tip 3 joined to a distal end of the catheter body 5 and made of a resin including tungsten powder 2, wherein the distal tip 3 includes a distal region 7 and a proximal region 9 disposed in a proximal side of the distal region 7, and a hardness of the distal region 7 is higher than a hardness of the proximal region 9, the proximal region 9 has a followability to a medical device such as a guidewire and the like used together, thus, the catheter 1 has an effect of improving insertability into the stenosis and an effect of preventing the collapse of the distal tip 3 of the catheter 1 when the catheter 1 is inserted into the stenosis.

### (Second Embodiment)

Next, a second embodiment of the present invention will be described. The drawings used in the present embodiment is also exaggerated to facilitate understanding of the present invention, the dimensions of the drawings differ from actual dimensions.

Hereinafter the second embodiment of the present invention will be described, the overall view of the catheter will not be described because it is similar to FIG 1. Portions common to the first embodiment will be denoted by the same reference numerals, descriptions of the portions will be omitted.

The catheter 11 of the second embodiment differs from the catheter 1 of the first embodiment in a distal tip configuration. That is, in the distal tip 3 of the catheter 1 of the first embodiment, powder of biocompatible metal or metal compound was mixed only in the distal region 7, not mixed in the proximal region 9.

In contrast, in the distal tip of the present embodiment, powder of biocompatible metal or metal compound is mixed in the distal region and the proximal region of the distal tip.

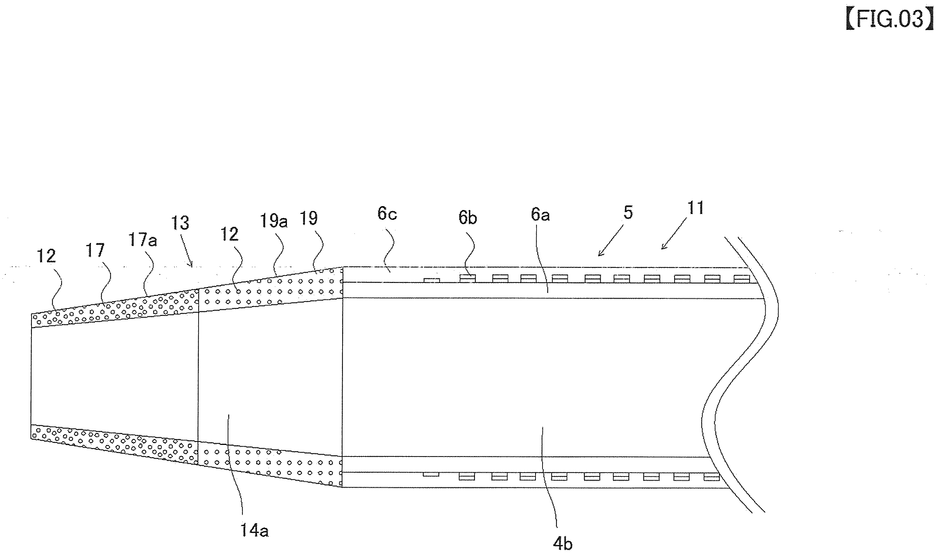

FIG. 3 is a longitudinal sectional view around a distal portion of the catheter of the second embodiment.

A catheter 11 of the present embodiment includes a catheter body 5 (corresponding to "a main body" of the present invention), a distal tip 3 (corresponding to "a distal portion" of the present invention) joined to a distal end of the catheter body 5, and a connector 8 joined to a proximal end of the catheter body 5.

The distal tip 13 is joined to the distal end of the catheter body 5 has a tubular shape with a lumen 14a communicating with the lumen 4b of the catheter body 5, and tapered periphery gradually decreasing diameter toward the distal end of the catheter body 5.

Further, the distal tip 13 includes a distal region 17 located on a distal side of the distal tip 13 and a proximal region 19 located on a proximal side of the distal region 17.

Resin material forming the distal tip 13 is not particularly limited, polyurethane elastomer is used in the present embodiment.

Powder made of biocompatible metal or metal compound is mixed inside the distal tip 13, tungsten powder 12 is mixed inside the distal tip 13 in the present embodiment.

In the present embodiment, tungsten powder 12 is mixed in the distal region 17 and the proximal region 19. The amount per unit volume of tungsten powder 12 in the distal region 17 is greater than the amount per unit volume of the tungsten powder 12 in the proximal region 19.

Thus, in the present embodiment, the hardness of the distal region 17 is set higher than the hardness of the proximal region 19. In addition, an outer surface 17a of the distal region 17 and an outer surface 19a of the proximal region 19 are smooth in the present embodiment.

According to the catheter 11 of the present embodiment, as the catheter 11 comprises a catheter body 5, and a distal tip 13 joined to a distal end of the catheter body 5 and made of a resin including tungsten powder 12, wherein the distal tip 13 includes a distal region 17 and a proximal region 19 disposed in a proximal side of the distal region 17, and a hardness of the distal region 17 is higher than a hardness of the proximal region 19, the proximal region 19 has a followability to a medical device such as a guidewire and the like used together, thus, the catheter 1 has an effect of improving insertability into the stenosis and an effect of preventing the collapse of the distal tip 13 of the catheter 11 when the catheter 11 is inserted into the stenosis.

### (Third Embodiment)

Next, a third embodiment of the present invention will be described. The drawings used in the present embodiment is also exaggerated to facilitate understanding of the present invention, the dimensions of the drawings differ from actual dimensions.

Hereinafter the third embodiment of the present invention will be described, the overall view of the catheter will not be described because it is similar to FIG 1. Portions common to the first embodiment will be denoted by the same reference numerals, descriptions of the portions will be omitted.

The catheter 21 of the third embodiment differs from the catheter 11 of the second embodiment in condition of an outer surface of the distal tip of the catheter. That is, an outer surface 17a and an outer surface 19a of the distal tip 13 of catheter 11 of the second embodiment are smooth. In contrast, an outer surface of the distal tip of the present embodiment is irregular.

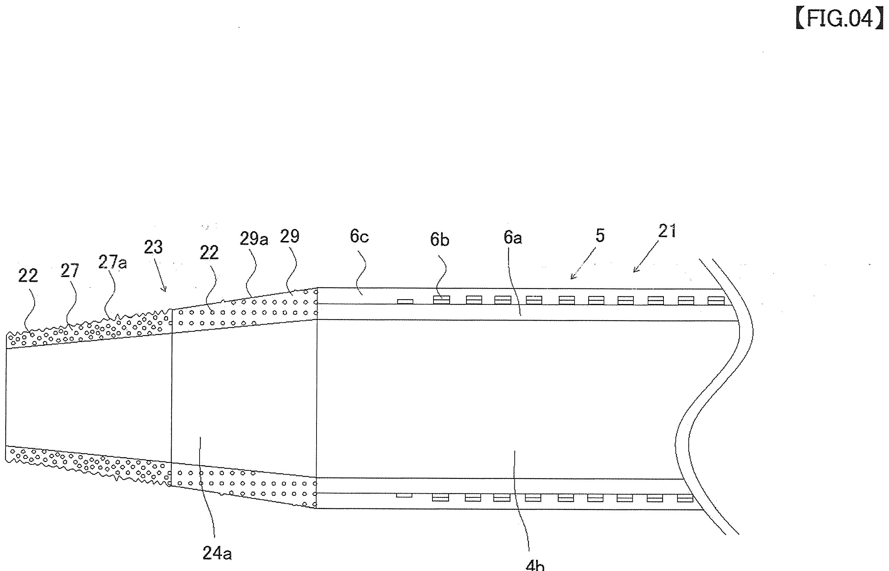

FIG. 4 is a longitudinal sectional view around a distal portion of the catheter of the third embodiment.

A catheter 21 of the present embodiment includes a catheter body 5 (corresponding to "a main body" of the present invention), a distal tip 23 (corresponding to "a distal portion" of the present invention) joined to a distal end of the catheter body 5, and a connector 8 joined to a proximal end of the catheter body 5.

The distal tip 23 is joined to the distal end of the catheter body 5 has a tubular shape with a lumen 24a communicating with the lumen 4b of the catheter body 5, and tapered periphery gradually decreasing diameter toward the distal end of the catheter body 5.

Further, the distal tip 23 includes a distal region 27 located on a distal side of the distal tip 23 and a proximal region 29 located on a proximal side of the distal region 27.

Resin material forming the distal tip 23 is not particularly limited, polyurethane elastomer is used in the present embodiment.

Powder made of biocompatible metal or metal compound is mixed inside the distal tip 23, tungsten powder 22 is mixed inside the distal tip 23 in the present embodiment.

In the present embodiment, tungsten powder 22 is mixed in the distal region 27 and the proximal region 29. The amount per unit volume of tungsten powder 22 in the distal region 27 is greater than the amount per unit volume of the tungsten powder 22 in the proximal region 29. Thus, in the present embodiment, the hardness of the distal region 27 is set higher than the hardness of the proximal region 29.

Further, in the present embodiment, a surface roughness of an outer surface 27a of the distal region 27 is greater than a surface roughness of the outer surface 29a of the proximal region 29. Incidentally, it is possible by known techniques such as a sandblasting to control the surface roughness of the distal tip 23.

According to the catheter 21 of the present embodiment, as the catheter 21 comprises a catheter body 5, and a distal tip 23 joined to a distal end of the catheter body 5 and made of a resin including tungsten powder 22, wherein the distal tip 23 includes a distal region 27 and a proximal region 29 disposed in a proximal side of the distal region 27, a hardness of the distal region 27 is higher than a hardness of the proximal region 29, and the surface roughness of the outer surface 27a of the distal region 27 is greater than the surface roughness of the outer surface 29a of proximal region 29, in addition to the effects of the catheter 1 of the first embodiment and the catheter 11 of the second embodiment, it is capable of further improving insertability into the stenosis to reduce the contact resistance between the distal region 27 having high-hardness and the stenosis.

### (Fourth Embodiment)

Next, a fourth embodiment of the present invention will be described. The drawings used in the present embodiment is also exaggerated to facilitate understanding of the present invention, the dimensions of the drawings differ from actual dimensions.

Hereinafter the fourth embodiment of the present invention will be described, the overall view of the catheter will not be described because it is similar to FIG 1. Portions common to the first embodiment will be denoted by the same reference numerals, descriptions of the portions will be omitted.

The catheter 31 of the fourth embodiment differs from the catheter 21 of the third embodiment in structure of an outer surface of the distal tip of the catheter. That is, an outer surface 27a and an outer surface 29a of the distal tip 23 of catheter 21 of the third embodiment were configured to form an irregular state by resin constituting the distal tip 23. In contrast, an outer surface of the distal tip of the present embodiment is configuired to form an irregular state by powder of biocompatible metal or metal compound mixed inside the distal chip 23.

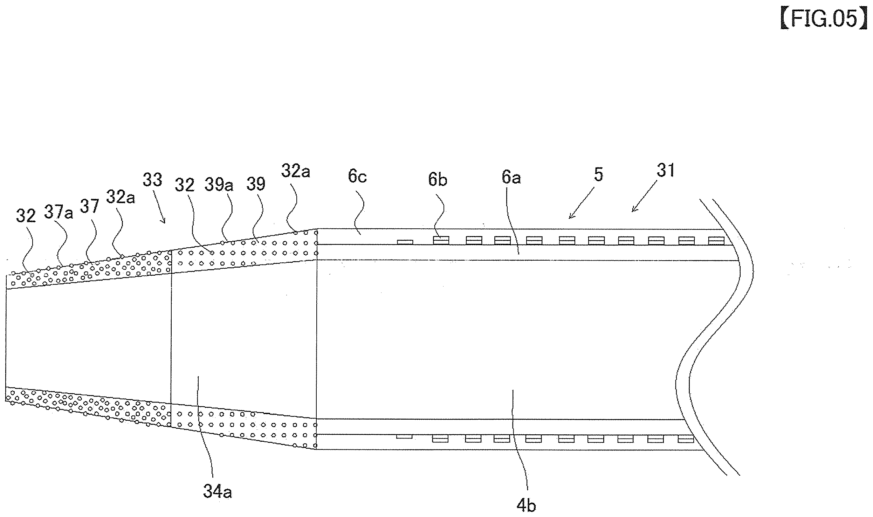

FIG. 5 is a longitudinal sectional view around a distal portion of the catheter of the fourth embodiment.

A catheter 31 of the present embodiment includes a catheter body 5 (corresponding to "a main body" of the present invention), a distal tip 33 (corresponding to "a distal portion" of the present invention) joined to a distal end of the catheter body 5, and a connector 8 joined to a proximal end of the catheter body 5.

The distal tip 33 is joined to the distal end of the catheter body 5 has a tubular shape with a lumen 34a communicating with the lumen 4b of the catheter body 5, and tapered periphery gradually decreasing diameter toward the distal end of the catheter body 5.

Further, the distal tip 33 includes a distal region 37 located on a distal side of the distal tip 33 and a proximal region 39 located on a proximal side of the distal region 37.

Resin material forming the distal tip 33 is not particularly limited, polyurethane elastomer is used in the present embodiment.

Powder made of biocompatible metal or metal compound is mixed inside the distal tip 33, tungsten powder 32 is mixed inside the distal tip 33 in the present embodiment.

In the present embodiment, tungsten powder 32 is mixed in the distal region 37 and the proximal region 39. The amount per unit volume of tungsten powder 32 in the distal region 37 is greater than the amount per unit volume of the tungsten powder 32 in the proximal region 39. Thus, in the present embodiment, the hardness of the distal region 37 is set higher than the hardness of the proximal region 39.

Further, in the present embodiment, a surface roughness of an outer surface 37a of the distal region 37 is greater than a surface roughness of the outer surface 39a of the proximal region 39.

A convex portion in the irregular state defining a surface roughness is formed by protrusion of tungsten 32 mixed inside the distal region 37 and the proximal region 39 from the outer surface 37a of the distal region 37 and the outer surface 39a of the proximal region 39 (a tungsten 32 protruding from the outer surface 39a is written as a projecting tungsten 32a in FIG.5) in the present embodiment.

In the present embodiment, as the amount per unit volume of tungsten powder 32 in the distal region 37 is larger than the amount per unit volume of tungsten powder 32 in the proximal region 39, the number per unit area of the projecting tungsten 32a on the outer surface 37a of the distal region 37 can be easily increased more than the number per unit area of the projecting tungsten 32a on the outer surface 39a of the proximal region 39 to remove resin constituting the distal tip 33 by means of simply melting, evaporating or the like.

In this case, degree of exposure of the tungsten 32a on the outer surface 37a of the distal region 37 is higher than degree of exposure of the tungsten 32a on the outer surface 39a of the proximal region 39.

According to the catheter 31 of the present embodiment, as the catheter 31 comprises a catheter body 5, and a distal tip 33 joined to a distal end of the catheter body 5 and made of a resin including tungsten powder 32, wherein the distal tip 33 includes a distal region 37 and a proximal region 39 disposed in a proximal side of the distal region 37, a hardness of the distal region 37 is higher than a hardness of the proximal region 39, and degree of exposure of tungsten 32a on the outer surface 37a of the distal region 37 is higher than degree of exposure of tungsten 32a on the outer surface 39a of the proximal region 39, in addition to the effects of the catheter 21 of the third embodiment, it is capable of further improving insertability into the stenosis to reduce the contact resistance between the distal region 37 having high-hardness and the stenosis easily.

### (Fifth Embodiment)

Next, a fifth embodiment of the present invention will be described. The drawings used in the present embodiment is also exaggerated to facilitate understanding of the present invention, the dimensions of the drawings differ from actual dimensions.

Hereinafter the fifth embodiment of the present invention will be described, the overall view of the catheter will not be described because it is similar to FIG 1. Portions common to the first embodiment will be denoted by the same reference numerals, descriptions of the portions will be omitted.

The catheter 41 of the fifth embodiment differs from the catheter 31 of the fourth embodiment in structure of a distal region of the distal tip of the catheter. That is, a distal region 32 of the distal tip 33 of the catheter 31 of the fourth embodiment is constituted by resin in normal state except tungsten 32. In contrast, a distal region of the distal tip of the present embodiment is constituted by evaporating a certain amount of resin from resin constituting the distal region 37.

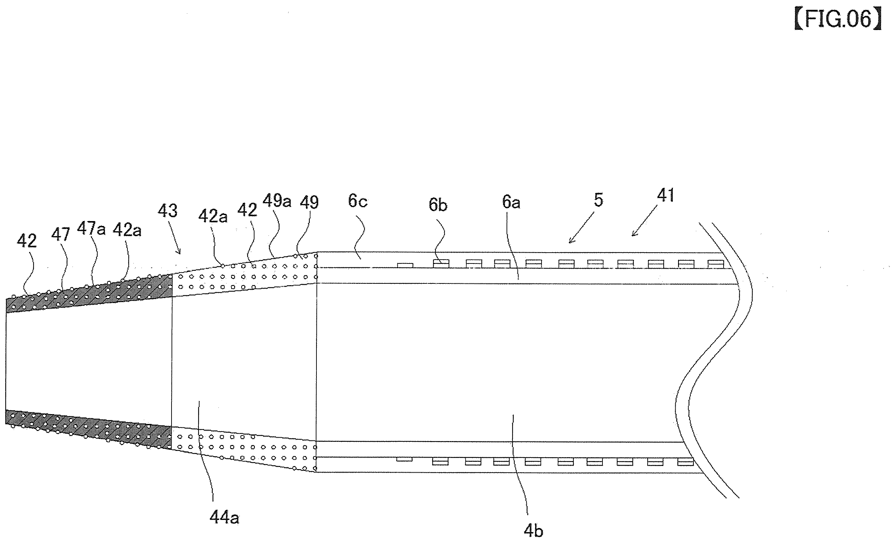

FIG. 6 is a longitudinal sectional view around a distal portion of the catheter of the fifth embodiment.

A catheter 41 of the present embodiment includes a catheter body 5 (corresponding to "a main body" of the present invention), a distal tip 43 (corresponding to "a distal portion" of the present invention) joined to a distal end of the catheter body 5, and a connector 8 joined to a proximal end of the catheter body 5.

The distal tip 43 is joined to the distal end of the catheter body 5 has a tubular shape with a lumen 44a communicating with the lumen 4b of the catheter body 5, and tapered periphery gradually decreasing diameter toward the distal end of the catheter body 5.

Further, the distal tip 43 includes a distal region 47 located on a distal side of the distal tip 43 and a proximal region 49 located on a proximal side of the distal region 47.

Resin material forming the distal tip 43 is not particularly limited, polyurethane elastomer is used in the present embodiment.

Powder made of biocompatible metal or metal compound is mixed inside the distal tip 43, tungsten powder 42 is mixed inside the distal tip 43 in the present embodiment.

In the present embodiment, tungsten powder 42 is mixed in the distal region 47 and the proximal region 49. The amount per unit volume of tungsten powder 42 in the distal region 47 is substantially equal to the amount per unit volume of the tungsten powder 42 in the proximal region 49.

On the other hand, in the distal region 47 of the distal tip 43 of the present embodiment, a certain amount of resin is evaporated from the outer surface 47a of the distal region 47 by laser irradiation such as excimer laser etc. Thus, the hardness of the distal region 47 is set higher than the hardness of the proximal region 49 in the present embodiment.

Further, in the present embodiment, a surface roughness of an outer surface 47a of the distal region 47 is greater than a surface roughness of the outer surface 49a of the proximal region 49. This is set by controlling irradiation time and the like of the excimer laser etc. so that evaporation amount of the resin in the distal region 47 is more than evaporation amount of the resin in the proximal region 49.

In the present embodiment, as in the fourth embodiment, a convex portion in the irregular state defining a surface roughness is formed by protrusion of tungsten 42 mixed inside the distal region 47 and the proximal region 49 from the outer surface 47a of the distal region 47 and the outer surface 49a of the proximal region 49 (a tungsten 42 protruding from the outer surface 39a is written as a projecting tungsten 42a in FIG.6).

That is, in the present embodiment, the protrusion height of the projection tungsten 42a on the outer surface 47a of the distal region 47 is higher than the protrusion height of the projection tungsten 42a on the outer surface 49a of the proximal region 49. Thus, the surface roughness of the outer surface becomes great.

According to the catheter 41 of the present embodiment, as the catheter 41 comprises a catheter body 5, and a distal tip 43 joined to a distal end of the catheter body 5 and made of a resin including tungsten powder 42, wherein the distal tip 43 includes a distal region 47 and a proximal region 49 disposed in a proximal side of the distal region 47, a hardness of the distal region 47 is higher than a hardness of the proximal region 49 by evaporating a certain amount of resin from the distal region 47, and a surface roughness of the outer surface 47a of the distal region 47 is greater than a surface roughness of the outer surface 49a of the proximal region 49 by control the height of the projection tungsten 42a that tungsten 42 mixed inside the distal tip 43 protrudes from the outer surface of the distal tip 43, in addition to the effects of the catheter 31 of the fourth embodiment, the catheter 41 can be easily set a hardness difference between the distal region 47 and the proximal region 49. The proximal region 49 also has a followability to a medical device such as a guidewire and the like used together, thus, the catheter 41 has an effect of improving insertability into the stenosis and an effect of preventing the collapse of the distal tip 43 of the catheter 41 when the catheter 41 is inserted into the stenosis.

The catheter 41 includes the distal region 47 characterized in that it is formed by evaporating the resin of a distal portion more than the proximal region 49. According to this catheter 41, as the distal region 47 is formed by evaporating the resin of the distal porion more than the proximal region 49, a manufacturer can easily set a hardness of the distal region 47 and a hardness of the proximal region 49 by only adjusting energy of laser etc, and can easily set a surface roughness of the distal region 47 and a surface roughness of the proximal region 49 by only adjusting energy of laser etc.

While catheters of the various embodiments of the present invention have been described in each of the foregoing embodiments as an example of catheters, such catheters are not limited to the foregoing embodiments. Any catheter can be implemented with various modifications within a scope not departing from a subject matter thereof.

For example, a hardness difference between the distal region and the proximal region in the first to fourth embodiments causes on the basis of the amount of biocompatible metal or metal compound exemplified by tungsten mixed inside the distal region and the proximal region. However, as shown in the fifth embodiment, it is possible to set higher a hardness of the distal region than a hardness of the proximal region by evaporating a certain amount of resin from the distal region in the first to fourth embodiments.

Catheters in the first to fifth embodiments have been described as the distal tip 3, 13, 23, 33 and 43 are applied to a catheter of the simplest configuration. However, they are not limited thereto. The distal tip 3, 13, 23, 33 and 43 are applicable to any medical catheter such as a balloon catheter and a multi-lumen catheter.

### [Description of the code]

1, 11, 21, 31, 41 ... catheter
2, 12, 22, 32, 42 ... tungsten (Examples of biocompatible metal or metal compound)
3, 13, 23, 33,43 ... distal tip
7, 17, 27, 37, 47 ... distal region
9, 19, 29, 39,49 ... proximal region
5 ... catheter body
8 ... connector
6a ... inner layer
6b ... braid
6c ... outer layer

## Claims

1. A catheter, comprising:
a main body; and
a distal portion joined to a distal end of the main body, and made of a resin including a metal or a metal compound, wherein
the distal portion includes a distal region and a proximal region disposed in a proximal side of the distal region, and
hardness of the distal region is higher than hardness of the proximal region.

2. A catheter according to Claim 1,
surface roughness of the distal region is greater than surface roughness of the proximal region.

3. A catheter according to Claim 1 or Claim 2,
degree of exposure of the metal or the metal compound on the surface of the distal region is higher than degree of exposure of the metal or the metal compound on the surface of the proximal region.

4. A catheter according to any one of Claims 1 to 3,
the metal or the metal compound includes radiopaque metal.
